# EUROPEAN PATENT APPLICATION

(11) **EP 4 545 120 A1**
(43) Date of publication of application: **30.04.2025**
(21) Application number: 23827511.9
(22) Date of filing: 21.06.2023
(51) Int. Cl.: A61M 5/44, A61M 5/30, A61M 5/315, A61M 5/32, A61M 5/31

(54) **DRUG INJECTION DEVICE**

(30) Priority: 22.06.2022 KR 20220076227; 29.09.2022 KR 20220124348
(71) Applicant: Seo, Suk Bae, Seoul 06732 (KR); Kim, Ja Young, Seoul 06732 (KR); Seo, Ho Joon, Seoul 06732 (KR)
(72) Inventor: Seo, Suk Bae, Seoul 06732 (KR); Kim, Ja Young, Seoul 06732 (KR); Seo, Ho Joon, Seoul 06732 (KR)
(74) Representative: dompatent von Kreisler Selting Werner - Partnerschaft von Patent- und Rechtsanwälten mbB
(86) International application number: PCT/KR2023/008593
(87) International publication number: WO 2023/249401

(57) **Abstract**

Disclosed is a drug injection device including a chemical solution chamber in which a chemical solution is stored, a discharge part which is provided in the chemical solution chamber and through which the chemical solution is discharged, and a laser supply part that supplies a pulsed laser to the chemical solution to generate bubbles, wherein the bubbles induce volume expansion of the chemical solution and are dissipated to generate shock waves that push the chemical solution to the discharge part.

## Description

### [TECHNICAL FIELD]

Embodiments of the present disclosure described herein relate to a drug injection device.

### [BACKGROUND ART]

A drug injection device is a device that efficiently injects the necessary amount of drugs into a human body by minimizing side effects occurring in the related art and maximizing a treatment effect of medicines when the medicines are used for treatment of diseases or wounds of the human body.

A general syringe, which is most commonly used as the drug injection device, may accurately and efficiently administer drugs, but is an object of fear of patients due to pain during injection, and infection due to wounds is concerned.

To solve this problem, a needleless syringe has been developed.

For example, the needleless syringe includes a pressure chamber in which working fluid is stored, a chemical solution chamber which is coupled to the pressure chamber and in which a chemical solution is stored, an elastic film interposed between the pressure chamber and the chemical solution chamber, an injection nozzle provided in the chemical solution chamber, and a thermal energy transfer unit that transfers thermal energy to the working fluid.

In the related art, the thermal energy transfer unit may transfer the thermal energy to the working fluid stored in the pressure chamber to generate bubbles, the elastic film may be stretched and deformed toward the chemical solution chamber due to shock waves generated as the bubbles are rapidly expanded and dissipated, and accordingly, a propulsive pressure is applied to the chemical solution stored in the chemical solution chamber so that the chemical solution may be discharged through the injection nozzle.

However, in the related art, the pressure chamber, the elastic film, the chemical solution chamber, the injection nozzle, and the thermal energy transfer unit are included, and thus a structure thereof is very complex.

### [DETAILED DESCRIPTION OF THE INVENTION]

### [TECHINICAL PROBLEM]

Embodiments of the present disclosure provide a drug injection device in which a pressure chamber and an elastic film are not required and a structure thereof may be simplified.

The aspects of the present disclosure are not limited to the aspects described above, and those skilled in the art will clearly understand other aspects not described from the following description.

### [TECHNICAL SOLUTION]

According to an embodiment, a drug injection device includes a chemical solution chamber in which a chemical solution is stored, a discharge part which is provided in the chemical solution chamber and through which the chemical solution is discharged, and a laser supply part that supplies a pulsed laser to the chemical solution to generate bubbles, wherein the bubbles induce volume expansion of the chemical solution and are dissipated to generate shock waves that push the chemical solution to the discharge part.

Further, the chemical solution may be a liquid, a sol, or a liquid material in which the bubbles are generated by the pulsed laser.

Further, the drug injection device may further include a chemical solution storage part that stores the chemical solution to be supplied to the chemical solution chamber and a check valve that allows the chemical solution stored in the chemical solution storage part to be transferred only to the chemical solution chamber, wherein the chemical solution stored in the chemical solution storage part may be injected into the chemical solution chamber through the check valve by a negative pressure generated in the chemical solution chamber after the chemical solution is discharged through the discharge part.

Further, the drug injection device may further include a temperature sensor that measures a temperature of the chemical solution stored in the chemical solution chamber, a flow rate sensor that measures a flow rate of the chemical solution discharged through the discharge part, a pressure sensor that measures a pressure of the chemical solution stored in the chemical solution chamber, and a controller that forcibly opens the check valve so that the chemical solution stored in the chemical solution storage part is supplied to the chemical solution chamber when at least one of the temperature value measured by the temperature sensor, the flow rate value measured by the flow rate sensor, and the pressure value measured by the pressure sensor exceeds a set reference value.

Further, the chemical solution may include a filler.

Further, the drug injection device may further include a chemical solution guide that is provided in the discharge part and guides the chemical solution discharged through the discharge part to be sprayed in the form of fine particles.

Further, the chemical solution guide may include a guide body that is provided in the discharge part and reduces a cross-sectional area of the discharge part and a guide groove formed in a spiral shape on an outer circumferential surface of the guide body, and the chemical solution discharged through the discharge part may flow along the guide groove to generate a vortex and may be sprayed in the form of fine particles.

Further, the drug injection device may further include a temperature adjusting part that heats or cools the chemical solution stored in the chemical solution chamber so that a temperature of the chemical solution stored in the chemical solution chamber is maintained at a set temperature.

Further, the temperature adjusting part may include a Peltier element that is provided in the chemical solution chamber and emits cold or heat and a current supply part that adjusts a direction of a current applied to the Peltier element so that the Peltier element emits cold or heat according to the set temperature.

Further, the chemical solution chamber may include an insulating material.

Further, the drug injection device may further include an auxiliary temperature adjusting part that is provided in the chemical solution storage part and heats or cools the chemical solution stored in the chemical solution storage part so that the chemical solution stored in the chemical solution storage part is maintained at the set temperature.

Further, the temperature adjusting part may include an auxiliary Peltier element that is provided in the chemical solution storage part and emits cold or heat and an auxiliary current supply part that adjusts a direction of a current applied to the auxiliary Peltier element so that the auxiliary Peltier element emits cold or heat according to the set temperature.

Further, the drug injection device may further include a plunger provided to reciprocate in the chemical solution chamber and approaching the discharge part to apply a pressure for discharging the chemical solution stored in the chemical solution chamber to the discharge part, a moving coil provided in the plunger, a first magnetic coil that is provided on one side of the chemical solution chamber and applies a magnetic force for bringing the moving coil closer to the discharge part, and a second magnetic coil that is provide on an opposite side of the chemical solution chamber and applies a magnetic force for spacing the moving coil apart from the discharge part.

Further, the drug injection device may further include a moving coil current applying part that applies a pulse current to the moving coil, a first magnetic coil current applying part that applies a pulse current to the first magnetic coil, and a second magnetic coil current applying part that applies a pulse current to the second magnetic coil.

### [ADVANTAGEOUS EFFECTS OF THE INVENTION]

In the drug injection device according to the embodiment of the present disclosure, a pressure chamber and an elastic film are not required, and thus a structure thereof may be simplified.

The effects of the present disclosure are not limited to the effects described above, and those skilled in the art will clearly understand other effects not described from the following description.

### [DESCRIPTION OF THE DRAWINGS]

FIG. 1 is a cross-sectional view illustrating a drug injection device according to a first embodiment of the present disclosure.
FIGS. 2 to 4 are cross-sectional views illustrating a process in which the drug injection device according to the first embodiment of the present disclosure injects a drug into a skin.
FIG. 5 is a cross-sectional view illustrating a drug injection device according to a second embodiment of the present disclosure.
FIG. 6 is a cross-sectional view illustrating a drug injection device according to a third embodiment of the present disclosure.
FIG. 7 is a cross-sectional view illustrating a drug injection device according to a fourth embodiment of the present disclosure.
FIG. 8 is a cross-sectional view illustrating a drug injection device according to a fifth embodiment of the present disclosure.
FIGS. 9 to 11 are cross-sectional views illustrating a process in which the drug injection device according to the fifth embodiment of the present disclosure injects a drug into a skin.
FIG. 12 is a cross-sectional view illustrating a drug injection device according to a sixth embodiment of the present disclosure.
FIG. 13 is a cross-sectional view illustrating a drug injection device according to a seventh embodiment of the present disclosure.
FIG. 14 is a cross-sectional view illustrating a drug injection device according to an eighth embodiment of the present disclosure.
FIG. 15 is a cross-sectional view illustrating a drug injection device according to a ninth embodiment of the present disclosure.
FIG. 16 is a cross-sectional view illustrating a state in which the drug injection device according to the ninth embodiment of the present disclosure discharges a drug.

### [BEST MODE]

Advantages and features of the present disclosure and a method of achieving the advantages and the features will become apparent with reference to embodiments described below in detail together with the accompanying drawings. However, the inventive concept is not limited to the embodiments described below but may be implemented in various forms, and the present embodiments merely make the disclosure of the inventive concept complete and are provided to completely inform the scope of the inventive concept to those skilled in the art to which the inventive concept belongs, and the inventive concept is merely defined by the scope of the appended claims.

Unless otherwise defined, all the terms (including technical and scientific terms) used herein may be used as meanings that may be commonly understood by those skilled in the art to which the inventive concept belongs. Further, terms defined in a commonly used dictionary are not interpreted ideally or excessively unless explicitly and specifically defined.

Prior to the description of the present disclosure, in various embodiments, components having the same configurations will be representatively described in a first embodiment using the same reference numerals, and in other embodiments, components having configurations different from those of the first embodiment will be described.

Hereinafter, embodiments of the inventive concept will be described in detail with reference to the accompanying drawings.

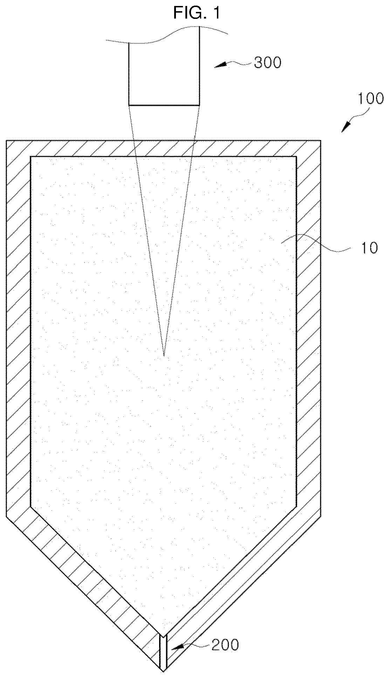

FIG. 1 is a cross-sectional view illustrating a drug injection device according to a first embodiment of the present disclosure.

As illustrated in FIG. 1, a drug injection device according to a first embodiment of the present disclosure may include a chemical solution chamber 100, a discharge part 200, and a laser supply part 300.

The chemical solution chamber 100 stores a chemical solution 10. In detail, the chemical solution chamber 100 may have a sealed storage space therein, and the chemical solution 10 may be stored in the storage space.

The chemical solution chamber 100 may be formed in a cylindrical shape. The chemical solution chamber 100 may be provided with the discharge part 200, which will be described below.

The discharge part 200 is provided in the chemical solution chamber 100 and discharges the chemical solution 10. Here, a driving principle in which the chemical solution 10 is discharged from the chemical solution chamber 100 through the discharge part 200 will be described below. For example, the discharge part 200 may have a linear shape, a spray nozzle shape, or a needle shape, but the present disclosure is not particularly limited thereto, and other shapes may be applied thereto.

The discharge part 200 may be formed in a hole shape at a lower portion of the chemical solution chamber 100. A drug discharge rate may be determined according to a diameter of the discharge part 200. For example, the diameter of the discharge part 200 may be in a range of 50 micrometers to 1000 micrometers. However, when the diameter of the discharge part 200 is less than 50 micrometers, the amount of chemical solution 10 discharged from the discharge part 200 is relatively small, and thus the chemical solution 10 may not be injected into a skin at a sufficient depth. Further, when the diameter of the discharge part 200 is greater than 1000 micrometers, the amount of chemical solution 10 discharged from the discharge part 200 is relatively large, and thus the amount of chemical solution 10 that may not be injected into the skin and may be bounced off may increase. Thus, it is preferable that the diameter of the discharge part 200 is limited to the above-described value.

The laser supply part 300 supplies a pulsed laser to the chemical solution 10 to generate bubbles 20. These bubbles 20 induce volume expansion of the chemical solution 10 and are dissipated to generate shock waves that push the chemical solution 10 to the discharge part 200, and as a result, the chemical solution 10 may be discharged from the chemical solution chamber 100 through the discharge part 200.

For example, the laser supply part 300 may supply the pulsed laser oscillating at 10 Hz to 40 Hz per second to the chemical solution 10.

Meanwhile, when the bubbles 20 occur and the volume expansion of the chemical solution 10 occurs, a positive pressure may be formed in the chemical solution chamber 100. Thereafter, the bubbles 20 are dissipated to generate the shock waves, the chemical solution 10 is discharged from the chemical solution chamber 100 through the discharge part 200, and then a negative pressure may be formed in the chemical solution chamber 100.

The chemical solution 10 used in the drug injection device according to the embodiment of the present disclosure may be a liquid, a sol, or a liquid material in which the bubbles 20 are generated by the pulsed laser. In other words, the chemical solution 10 may be a liquid, a sol, or a liquid material that absorbs the pulsed laser, breaks down a liquid molecular structure, and generates the bubbles. For example, the chemical solution 10 may be a filler or water. Here, the filler may be a polymer-based filler, specifically, a polylactic acid (PLA), a poly-DL-lactic acid (PDLA), a polycaprolactone (PCL), or a polydioxanone (PDO).

In the embodiment, when the chemical solution 10 stored in the chemical solution chamber 100 is the polymer-based filler, even when the pulsed laser is instantaneously applied to the polymer-based filler, a temperature of the polymer-based filler easily increases and the polymer-based filler becomes soft due to characteristics of the polymer-based filler, the polymer-based filler is quickly broken down, and thus the polymer-based filler may be easily injected into the skin to provide a collagen regeneration effect to internal tissues of the skin.

Hereinafter, a process in which the drug injection device according to the first embodiment of the present disclosure injects the drug into the skin will be described. A controller 600 may perform the following process. Further, the following process may be applied to a second embodiment, a third embodiment, and a fourth embodiment.

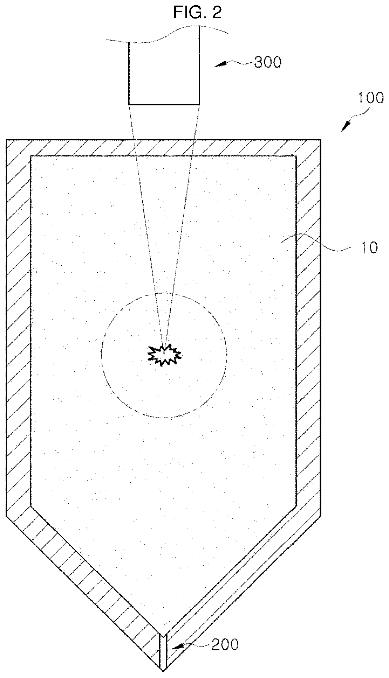

FIGS. 2 to 4 are cross-sectional views illustrating a process in which the drug injection device according to the first embodiment of the present disclosure injects a drug into a skin.

First, as illustrated in FIG. 2, the laser supply part 300 supplies the pulsed laser to a specific point of the chemical solution 10 filled in the chemical solution chamber 100.

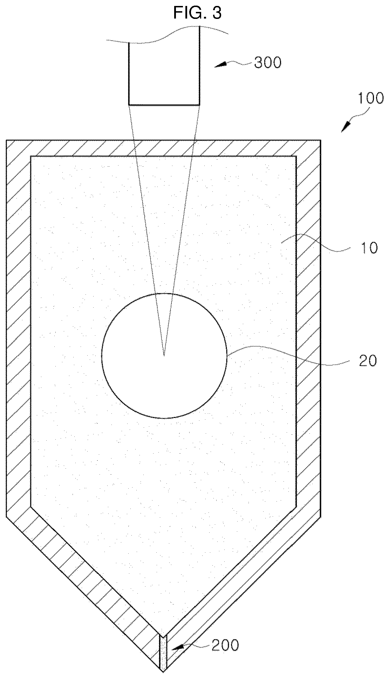

Next, as illustrated in FIG. 3, a molecular structure of the chemical solution 10 that absorbs the pulsed laser is broken down, and the bubbles 20 are generated.

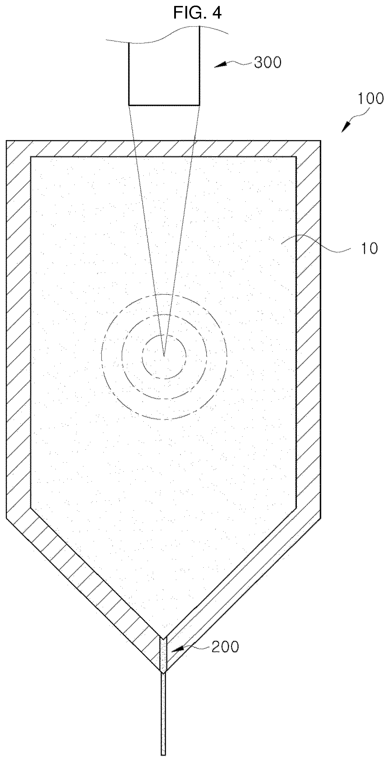

Next, as illustrated in FIG. 4, the bubbles 20 generated in the chemical solution chamber 100 induce the volume expansion of the chemical solution 10 and are dissipated to generate the shock waves that push the chemical solution 10 to the discharge part 200. As a result, the chemical solution 10 may be discharged from the chemical solution chamber 100 to the discharge part 200.

In the related art, a thermal energy transfer unit transfers thermal energy to working fluid stored in a pressure chamber to generate bubbles, an elastic film is stretched and deformed toward a chemical solution chamber by shock waves generated as the bubbles are quickly expanded and dissipated, and accordingly, a propulsive pressure is applied to a chemical solution stored in the chemical solution chamber, and the chemical solution is discharged through an injection nozzle. The drug injection device includes the pressure chamber, the elastic film, the chemical solution chamber, the injection nozzle, and the thermal energy transfer unit and thus has a very complex structure.

On the other hand, in the drug injection device according to the first embodiment of the present disclosure, even without the pressure chamber and the elastic film, the laser supply part 300 supplies the pulsed laser directly to the chemical solution 10 stored in the chemical solution chamber 100 to generate the bubbles 20, and the chemical solution 10 stored in the chemical solution chamber 100 is discharged by the shock waves generated as the bubbles 20 induce the volume expansion of the chemical solution 10 and are dissipated. The pressure chamber and the elastic film are not required, and thus a structure may be simplified.

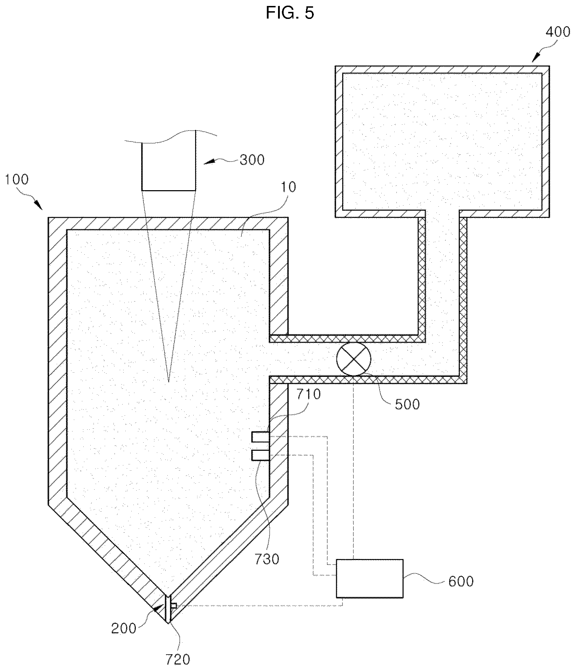

FIG. 5 is a cross-sectional view illustrating a drug injection device according to a second embodiment of the present disclosure.

As illustrated in FIG. 5, the drug injection device according to the second embodiment of the present disclosure may further include a chemical solution storage part 400, a check valve 500, and the controller 600, which is unlike the first embodiment.

The chemical solution storage part 400 stores the chemical solution 10 to be supplied to the chemical solution chamber 100. For example, a chemical solution tank in which the chemical solution 10 is stored may be used as the chemical solution storage part 400.

The check valve 500 may allow the chemical solution 10 stored in the chemical solution storage part 400 to be transferred only to the chemical solution chamber 100.

Thus, in the present embodiment, the chemical solution 10 stored in the chemical solution storage part 400 may be injected into the chemical solution storage part 400 through the check valve 500 by a negative pressure generated in the chemical solution chamber 100 after the chemical solution 10 is discharged through the discharge part 200.

The drug injection device according to the second embodiment of the present disclosure may further include a temperature sensor 710, a flow rate sensor 720, a pressure sensor 730, and the controller 600.

The temperature sensor 710 may measure a temperature of the chemical solution 10 stored in the chemical solution chamber 100. The temperature sensor 710 may be provided in the chemical solution chamber 100.

The flow rate sensor 720 may measure a flow rate of the chemical solution 10 discharged through the discharge part 200. The flow rate sensor 720 may be provided in the discharge part 200.

The pressure sensor 730 may measure a pressure of the chemical solution 10 stored in the chemical solution chamber 100. The pressure sensor 730 may be provided in the chemical solution chamber 100.

When at least one of the temperature value measured by the temperature sensor 710, the flow rate value measured by the flow rate sensor 720, and the pressure value measured by the pressure sensor 730 exceeds a set reference value, the controller 600 may forcibly open the check valve 500 so that the chemical solution 10 stored in the chemical solution storage part 400 is supplied to the chemical solution chamber 100. Here, in the controller 600, the set reference value for the temperature value may be in a range of 37 degrees to 40 degrees, the set reference value for the flow rate value may be in a range of 3 ml to 4 ml, and the set reference value for the pressure value may be 1 atmosphere.

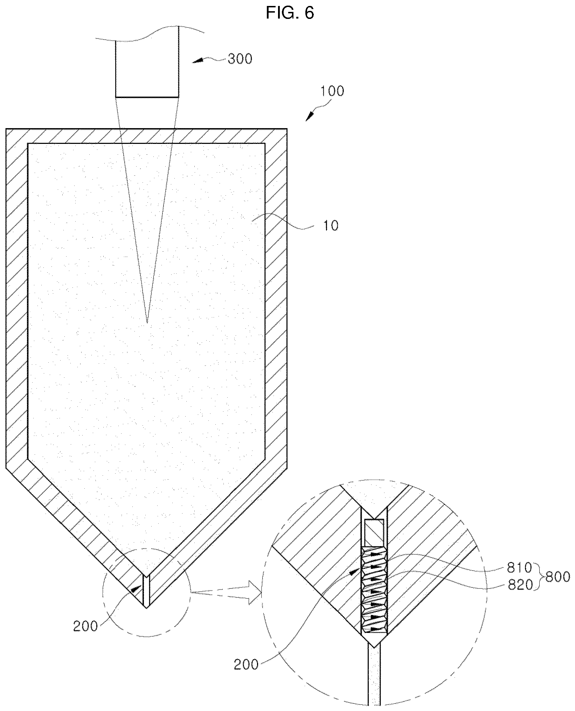

FIG. 6 is a cross-sectional view illustrating a drug injection device according to a third embodiment of the present disclosure.

As illustrated in FIG. 6, the drug injection device according to the third embodiment of the present disclosure may further include a chemical solution guide 800, which is unlike the first embodiment.

The chemical solution guide 800 may be provided in the discharge part 200 to guide the chemical solution 10 discharged through the discharge part 200 to be sprayed in the form of fine particles. The chemical solution guide 800 may include a guide body 810 and a guide groove 820.

The guide body 810 is provided in the discharge part 200 and serves to reduce a cross-sectional area of the discharge part 200.

The guide groove 820 may be formed in a spiral shape on an outer circumferential surface of the guide body 810.

Thus, the chemical solution 10 discharged through the discharge part 200 may flow along the guide groove 820 to generate a vortex and thus may be sprayed in the form of fine particles.

The fine particles sprayed through the discharge part 200 may have a very small diameter and a very slow flow rate and thus may easily pass through a skin surface and be easily injected into a deep skin.

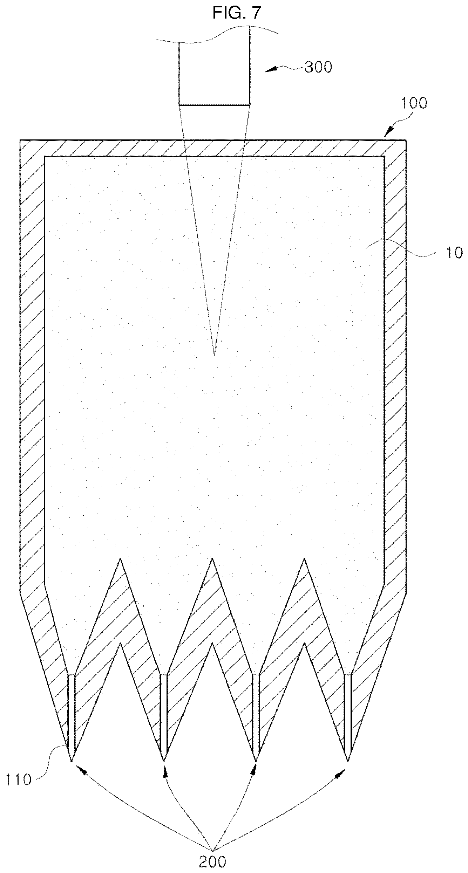

FIG. 7 is a cross-sectional view illustrating a drug injection device according to a fourth embodiment of the present disclosure.

As illustrated in FIG. 7, in the drug injection device according to the fourth embodiment of the present disclosure, unlike the first embodiment, a plurality of protrusions 110 may be formed at lower portions of the chemical solution chamber 100, and the plurality of discharge parts 200 may be provided in the plurality of protrusions 110, respectively.

The protrusion 110 may have a shape of which a diameter decreases as it approaches the skin.

Thus, in the present embodiment, as the drug is discharged through the plurality of discharge parts 200, a drug injection range of the skin may be expanded.

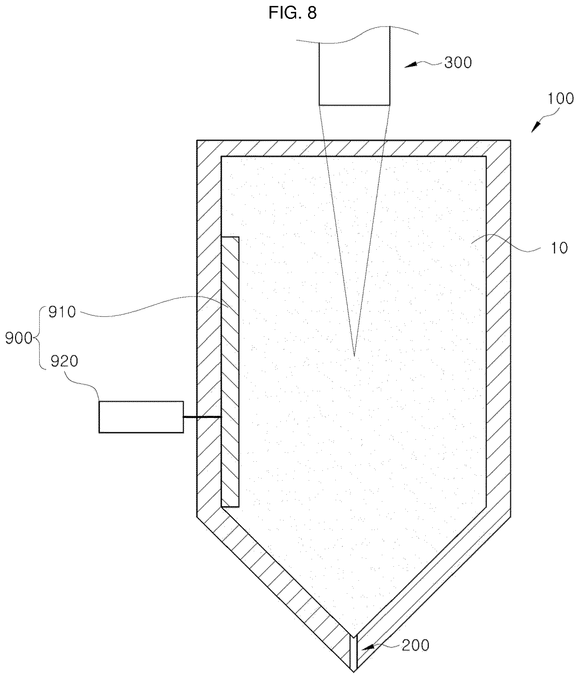

FIG. 8 is a cross-sectional view illustrating a drug injection device according to a fifth embodiment of the present disclosure.

As illustrated in FIG. 8, the drug injection device according to the fifth embodiment of the present disclosure may include the chemical solution chamber 100, the discharge part 200, the laser supply part 300, and a temperature adjusting part 900.

The chemical solution chamber 100 stores the chemical solution 10. In detail, the chemical solution chamber 100 may have the sealed storage space therein, and the chemical solution 10 may be stored in the storage space.

The chemical solution chamber 100 may be formed in a cylindrical shape. The chemical solution chamber 100 may be provided with the discharge part 200, which will be described below.

In the embodiment, the chemical solution chamber 100 may be maintained at a set temperature by the temperature adjusting part 900, which will be described below. In this case, the chemical solution chamber 100 may include an insulating material so that heat loss of the chemical solution chamber 100 is minimized. For example, the insulating material may include styrofoam or the like.

The discharge part 200 is provided in the chemical solution chamber 100 and discharges the chemical solution 10. Here, a driving principle in which the chemical solution 10 is discharged from the chemical solution chamber 100 through the discharge part 200 will be described below. For example, the discharge part 200 may have a linear shape, a spray nozzle shape, or a needle shape, but the present disclosure is not particularly limited thereto, and other shapes may be applied thereto.

The discharge part 200 may be formed in a hole shape at a lower portion of the chemical solution chamber 100. The drug discharge rate may be determined according to the diameter of the discharge part 200. For example, the diameter of the discharge part 200 may be in a range of 50 micrometers to 1000 micrometers. However, when the diameter of the discharge part 200 is less than 50 micrometers, the amount of chemical solution 10 discharged from the discharge part 200 is relatively small, and thus the chemical solution 10 may not be injected into a skin at a sufficient depth. Further, when the diameter of the discharge part 200 is greater than 1000 micrometers, the amount of chemical solution 10 discharged from the discharge part 200 is relatively large, and thus the amount of chemical solution 10 that may not be injected into the skin and may be bounced off may increase. Thus, it is preferable that the diameter of the discharge part 200 is limited to the above-described value.

The laser supply part 300 supplies the pulsed laser to the chemical solution 10 to generate the bubbles 20. These bubbles 20 induce the volume expansion of the chemical solution 10 and are dissipated to generate the shock waves that push the chemical solution 10 to the discharge part 200, and as a result, the chemical solution 10 may be discharged from the chemical solution chamber 100 through the discharge part 200. In this case, the chemical solution 10 discharged through the discharge part 200 may be injected into the deep skin.

For example, the laser supply part 300 may supply the pulsed laser oscillating at 10 Hz to 40 Hz per second to the chemical solution 10.

Meanwhile, when the bubbles 20 occur and the volume expansion of the chemical solution 10 occurs, a positive pressure may be formed in the chemical solution chamber 100. Thereafter, the bubbles 20 are dissipated to generate the shock waves, the chemical solution 10 is discharged from the chemical solution chamber 100 through the discharge part 200, and then a negative pressure may be formed in the chemical solution chamber 100.

The temperature adjusting part 900 may serve to heat or cool the chemical solution 10 stored in the chemical solution chamber 100 so that the temperature of the chemical solution 10 stored in the chemical solution chamber 100 is maintained at the set temperature. Thus, as the temperature of the chemical solution 10 stored in the chemical solution chamber 100 is maintained at the set temperature by the temperature adjusting part 900, the chemical solution 10 discharged through the discharge part 200 and injected into the deep skin may transfer heat or cold to the deep skin.

In the embodiment, the temperature adjusting part 900 may heat or cool the chemical solution chamber 100 so that the temperature of the chemical solution 10 stored in the chemical solution chamber 100 is maintained at the set temperature based on the temperature of the chemical solution 10, which is measured by the temperature sensor 710, which will be described below. Further, the temperature adjusting part 900 may be controlled by the controller 600, which will be described below.

In another embodiment, the set temperature of the temperature adjusting part 900 may be in a range of -10 °C to 50 °C.

For example, when the set temperature of the temperature adjusting part 900 is in a range of -10 °C to 40 °C, the chemical solution 10 discharged through the discharge part 200 and injected into the deep skin may transfer cold to the deep skin. In this case, when the set temperature of the temperature adjusting part 900 is in a range of -10 °C to 0 °C, an anti-freezer such as a glycerin solution is mixed with the chemical solution 10 to prevent the chemical solution 10 from freezing. Furthermore, when the chemical solution 10 having a temperature of -10 °C to 0 °C is injected into the deep skin, the chemical solution 10 may be used to treat keloids, raised skins, and skin scars in the deep skin.

Further, when the set temperature of the temperature adjusting part 900 is in a range of 40 °C to 50 °C, the chemical solution 10 discharged through the discharge part 200 and injected into the deep skin may transfer heat to the deep skin.

The temperature adjusting part 900 may include a Peltier element 910 and a current supply part 920.

The Peltier element 910 is provided in the chemical solution chamber 100 and serves to discharge cold or heat. For example, the Peltier element 910 may discharge cold on one surface thereof and discharge heat on an opposite surface thereof when a current is applied in a forward direction and may discharge heat on the one surface thereof and discharge heat on the opposite surface thereof when the current is applied in a reverse direction. Referring to the present example, the one surface of the Peltier element 910 may be in contact with the chemical solution 10 stored in the chemical solution chamber 100, and the opposite surface thereof may be coupled to an inner surface of the chemical solution chamber 100. When the current is applied to the Peltier element 910 in the forward direction, cold may be discharged from the one surface of the Peltier element 910 into the chemical solution 10 stored in the chemical solution chamber 100. Further, when the current is applied to the Peltier element 910 in the reverse direction, heat may be discharged from the one surface of the Peltier element 910 into the chemical solution 10 stored in the chemical solution chamber 100.

The current supply part 920 may adjust a direction of the current applied to the Peltier element 910 so that the Peltier element 910 emits cold or heat according to the set temperature. As in the example described above, when the one surface of the Peltier element 910 is in contact with the chemical solution chamber 100 and the opposite surface of the Peltier element 910 is not in contact with the chemical solution chamber 100, when the current supply part 920 applies the current to the Peltier element 910 in the forward direction, cold may be discharged from the one surface of the Peltier element 910 into the chemical solution 10 stored in the chemical solution chamber 100, and when the current supply part 920 applies the current to the Peltier element 910 in the reverse direction, heat may be discharged from the one surface of the Peltier element 910 into the chemical solution 10 stored in the chemical solution chamber 100.

The chemical solution 10 used in the drug injection device according to the embodiment of the present disclosure may be a liquid, a sol, or a liquid material in which the bubbles 20 are generated by the pulsed laser. In other words, the chemical solution 10 may be a liquid, a sol, or a liquid material that absorbs the pulsed laser, breaks down the liquid molecular structure, and generates the bubbles. For example, the chemical solution 10 may be a filler or water. Here, the filler may be a polymer-based filler, specifically, a polylactic acid (PLA), a poly-DL-lactic acid (PDLA), a polycaprolactone (PCL), or a polydioxanone (PDO).

In the embodiment, when the chemical solution 10 stored in the chemical solution chamber 100 is the polymer-based filler, even when the pulsed laser is instantaneously applied to the polymer-based filler, a temperature of the polymer-based filler easily increases and the polymer-based filler becomes soft, the polymer-based filler is quickly broken down, and thus the polymer-based filler may be easily injected into the skin to provide the collagen regeneration effect to the internal tissues of the skin.

Hereinafter, a process in which the drug injection device according to the fifth embodiment of the present disclosure injects the drug into the skin will be described. The controller 600 may perform the following process. Further, the following process may be applied to a sixth embodiment, a seventh embodiment, and an eighth embodiment.

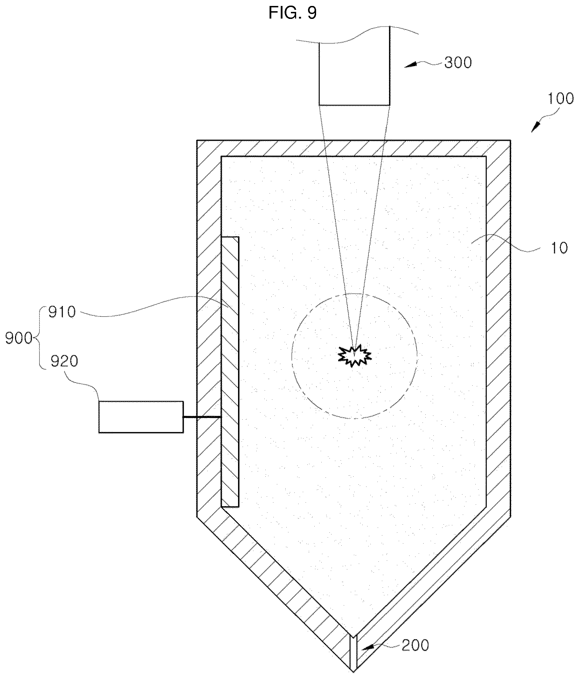

FIGS. 9 to 11 are cross-sectional views illustrating a process in which the drug injection device according to the fifth embodiment of the present disclosure injects a drug into a skin.

First, the temperature adjusting part 900 heats or cools the chemical solution 10 stored in the chemical solution chamber 100 so that the temperature of the chemical solution 10 stored in the chemical solution chamber 100 is maintained at the set temperature. For example, when the set temperature is higher than the measured temperature of the chemical solution 10 stored in the chemical solution chamber 100, the temperature adjusting part 900 may heat the chemical solution chamber 100. Further, when the set temperature is lower than the measured temperature of the chemical solution 10 stored in the chemical solution chamber 100, the temperature adjusting part 900 may cool the chemical solution chamber 100. In this case, an auxiliary temperature adjusting part 1000, which will be described below, may heat or cool the chemical solution 10 stored in the chemical solution storage part 400 so that the temperature of the chemical solution 10 stored in the chemical solution storage part 400, which will be described later, is maintained at the set temperature.

Thereafter, as illustrated in FIG. 9, the laser supply part 300 supplies the pulsed laser to a specific point of the chemical solution 10 filled in the chemical solution chamber 100.

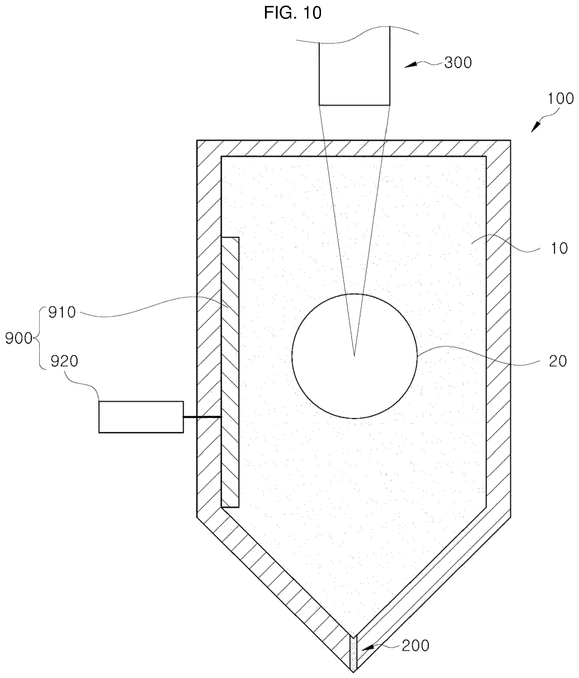

Next, as illustrated in FIG. 10, the molecular structure of the chemical solution 10 that absorbs the pulsed laser is broken down, and the bubbles 20 are generated.

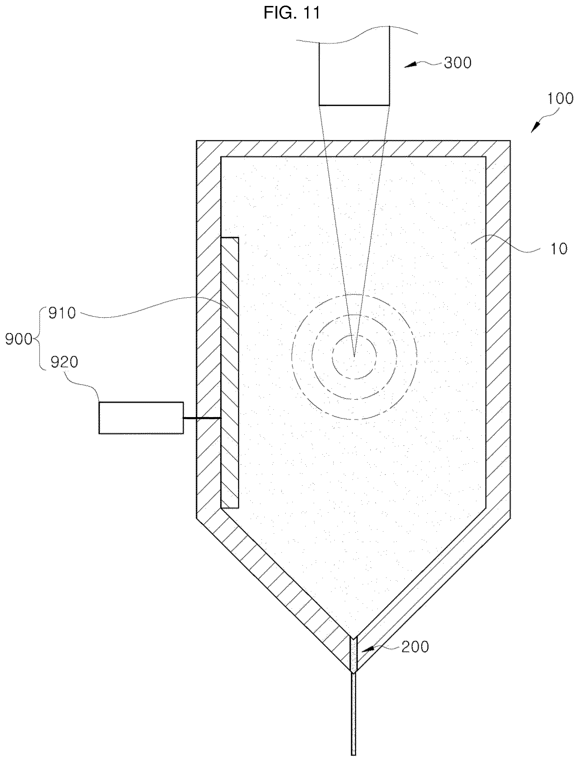

Next, as illustrated in FIG. 11, the bubbles 20 generated in the chemical solution chamber 100 induce the volume expansion of the chemical solution 10 and are dissipated to generate the shock waves that push the chemical solution 10 to the discharge part 200. As a result, the chemical solution 10 may be discharged from the chemical solution chamber 100 to the discharge part 200. The discharged chemical solution 10 may be injected into the deep skin to transfer heat or cold to the deep skin.

In the related art, a thermal energy transfer unit transfers thermal energy to working fluid stored in a pressure chamber to generate bubbles, an elastic film is stretched and deformed toward a chemical solution chamber by shock waves generated as the bubbles are quickly expanded and dissipated, and accordingly, a propulsive pressure is applied to a chemical solution stored in the chemical solution chamber, and the chemical solution is discharged through an injection nozzle. The drug injection device includes the pressure chamber, the elastic film, the chemical solution chamber, the injection nozzle, and the thermal energy transfer unit and thus has a very complex structure.

On the other hand, in the drug injection device according to the fifth embodiment of the present disclosure, even without the pressure chamber and the elastic film, the laser supply part 300 supplies the pulsed laser directly to the chemical solution 10 stored in the chemical solution chamber 100 to generate the bubbles 20, and the chemical solution 10 stored in the chemical solution chamber 100 is discharged by the shock waves generated as the bubbles 20 induce the volume expansion of the chemical solution 10 and are dissipated. The pressure chamber and the elastic film are not required, and thus a structure may be simplified.

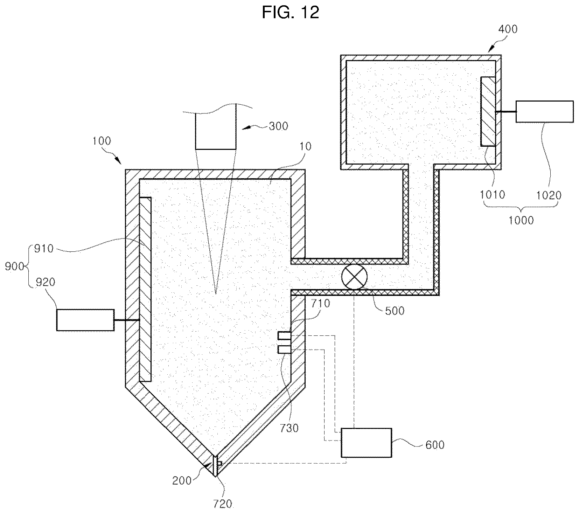

FIG. 12 is a cross-sectional view illustrating a drug injection device according to a sixth embodiment of the present disclosure.

As illustrated in FIG. 12, the drug injection device according to the sixth embodiment of the present disclosure may further include the chemical solution storage part 400, the check valve 500, and the controller 600, which is unlike the fifth embodiment.

The chemical solution storage part 400 stores the chemical solution 10 to be supplied to the chemical solution chamber 100. For example, the chemical solution tank in which the chemical solution 10 is stored may be used as the chemical solution storage part 400.

The check valve 500 may allow the chemical solution 10 stored in the chemical solution storage part 400 to be transferred only to the chemical solution chamber 100.

Thus, in the present embodiment, the chemical solution 10 stored in the chemical solution storage part 400 may be injected into the chemical solution chamber 100 through the check valve 500 by the negative pressure generated in the chemical solution chamber 100 after the chemical solution 10 is discharged through the discharge part 200.

The drug injection device according to the sixth embodiment of the present disclosure may further include the temperature sensor 710, the flow rate sensor 720, the pressure sensor 730, and the controller 600.

The temperature sensor 710 may measure the temperature of the chemical solution 10 stored in the chemical solution chamber 100. The temperature sensor 710 may be provided in the chemical solution chamber 100.

The flow rate sensor 720 may measure the flow rate of the chemical solution 10 discharged through the discharge part 200. The flow rate sensor 720 may be provided in the discharge part 200.

The pressure sensor 730 may measure the pressure of the chemical solution 10 stored in the chemical solution chamber 100. The pressure sensor 730 may be provided in the chemical solution chamber 100.

When at least one of the temperature value measured by the temperature sensor 710, the flow rate value measured by the flow rate sensor 720, and the pressure value measured by the pressure sensor 730 exceeds a set reference value, the controller 600 may forcibly open the check valve 500 so that the chemical solution 10 stored in the chemical solution storage part 400 is supplied to the chemical solution chamber 100. Here, in the controller 600, the set reference value for the temperature value may be in a range of 37 degrees to 40 degrees, the set reference value for the flow rate value may be in a range of 3 ml to 4 ml, and the set reference value for the pressure value may be 1 atmosphere.

Further, the drug injection device according to the sixth embodiment of the present disclosure may further include the auxiliary temperature adjusting part 1000.

The auxiliary temperature adjusting part 1000 may be provided in the chemical solution storage part 400 to heat or cool the chemical solution 10 stored in the chemical solution storage part 400 so that the temperature of the chemical solution 10 stored in the chemical solution storage part 400 is maintained at the set temperature.

Further, the auxiliary temperature adjusting part 1000 may include an auxiliary Peltier element 1010 and an auxiliary current supply part 1020.

The auxiliary Peltier element 1010 may be provided in the chemical solution storage part 400 to emit cold or heat. An operating principle of the auxiliary Peltier element 1010 may be the same as that of the Peltier element 910, except that an application target is the chemical solution storage part 400.

The auxiliary current supply part 1020 may adjust a direction of a current applied to the auxiliary Peltier element 1010 so that the auxiliary Peltier element 1010 emits cold or heat according to the set temperature. An operating principle of the auxiliary current supply part 1020 may be the same as that of the current supply part 920 except that an application target is the auxiliary Peltier element 1010.

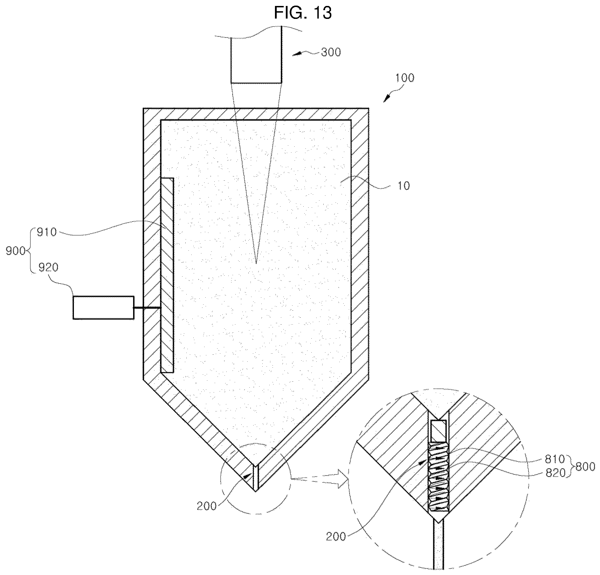

FIG. 13 is a cross-sectional view illustrating a drug injection device according to a seventh embodiment of the present disclosure.

As illustrated in FIG. 13, the drug injection device according to the seventh embodiment of the present disclosure may further include the chemical solution guide 800, which is unlike the fifth embodiment.

The chemical solution guide 800 may be provided in the discharge part 200 to guide the chemical solution 10 discharged through the discharge part 200 to be sprayed in the form of fine particles. The chemical solution guide 800 may include the guide body 810 and the guide groove 820.

The guide body 810 is provided in the discharge part 200 and serves to reduce the cross-sectional area of the discharge part 200.

The guide groove 820 may be formed in a spiral shape on the outer circumferential surface of the guide body 810.

Thus, the chemical solution 10 discharged through the discharge part 200 may flow along the guide groove 820 to generate a vortex and thus may be sprayed in the form of fine particles.

The fine particles sprayed through the discharge part 200 may have a very small diameter and a very slow flow rate and thus may easily pass through the skin surface and be easily injected into the deep skin.

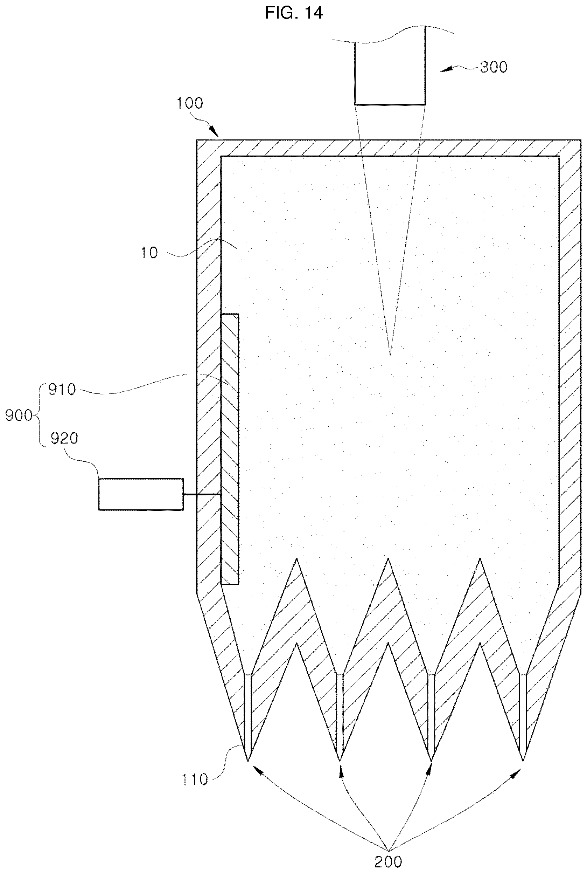

FIG. 14 is a cross-sectional view illustrating a drug injection device according to an eighth embodiment of the present disclosure.

As illustrated in FIG. 14, in the drug injection device according to the eighth embodiment of the present disclosure, unlike the fifth embodiment, the plurality of protrusions 110 may be formed at lower portions of the chemical solution chamber 100, and the plurality of discharge parts 200 may be provided in the plurality of protrusions 110, respectively.

The protrusion 110 may have a shape of which a diameter decreases as it approaches the skin.

Thus, in the present embodiment, as the drug is discharged through the plurality of discharge parts 200, the drug injection range of the skin may be expanded.

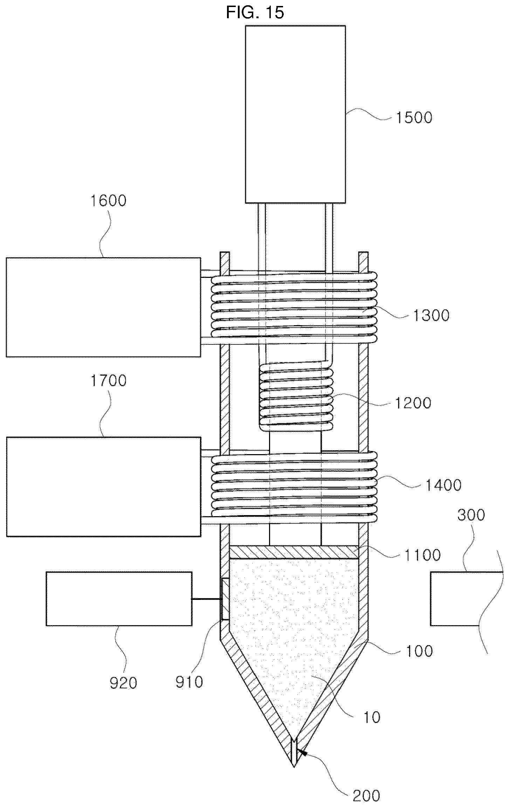

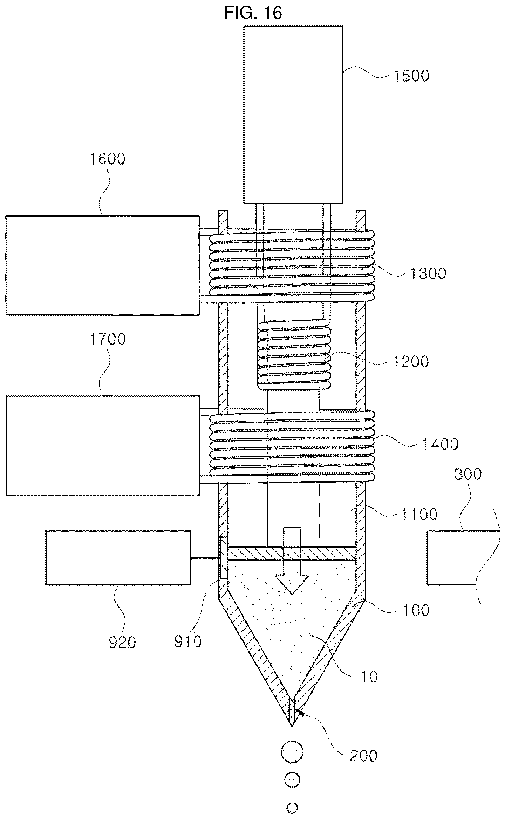

FIG. 15 is a cross-sectional view illustrating a drug injection device according to a ninth embodiment of the present disclosure, and FIG. 16 is a cross-sectional view illustrating a state in which the drug injection device according to the ninth embodiment of the present disclosure discharges a drug.

As illustrated in FIGS. 15 to 16, the drug injection device according to the ninth embodiment of the present disclosure may further include a plunger 1100, a moving coil 1200, a first magnetic coil 1300, a second magnetic coil 1400, a moving coil current applying part 1500, a first magnetic coil current applying part 1600, and a second magnetic coil current applying part 1700, which is unlike the fifth embodiment.

The plunger 1100 may be provided to reciprocate in the chemical solution chamber 100 and may approach the discharge part 200 to apply a pressure for discharging the chemical solution 10 stored in the chemical solution chamber 100 to the discharge part 200.

The plunger 1100 may include a plunger head and a plunger shaft. Here, the plunger head may have an outer diameter corresponding to an inner diameter of the chemical solution chamber 100, and the plunger shaft may have an outer diameter smaller than that of the plunger head.

The moving coil 1200 may be provided in the plunger 1100. The moving coil 1200 may move in a direction approaching the discharge part 200 by a magnetic force generated between the first magnetic coil 1300 and the moving coil 1200 and may move in a direction spaced apart from the discharge part 200 by a magnetic force generated between the second magnetic coil 1400 and the moving coil 1200, and details thereof will be described below. The moving coil 1200 may form a magnetic force by receiving a pulse current from the moving coil current applying part 1500, which will be described below.

The first magnetic coil 1300 may be provided on one side of the chemical solution chamber 100 to apply a magnetic force for bringing the moving coil 1200 closer to the discharge part 200. For example, the first magnetic coil 1300 may be provided at an upper end of the chemical solution chamber 100 at which there is no discharge part 200, and the moving coil 1200 may be provided between the first magnetic coil 1300 and the discharge part 200.

In the embodiment, the moving coil 1200 and the first magnetic coil 1300 may have a magnetic force having the same polarity when receiving the pulse current. Thus, when a magnetic force is simultaneously formed in the moving coil 1200 and the first magnetic coil 1300, a repulsive force may be generated between the moving coil 1200 and the first magnetic coil 1300, and thus the moving coil 1200 and the plunger 1100 may be moved in a direction approaching the chemical solution chamber 100.

The second magnetic coil 1400 may be provided on an opposite side of the chemical solution chamber 100 to apply a magnetic force for spacing the moving coil 1200 apart from the discharge part 200. For example, the second magnetic coil 1400 may be provided at a lower end of the chemical solution chamber 100 at which there is the discharge part 200, and the moving coil 1200 may be provided between the first magnetic coil 1300 and the second magnetic coil 1400.

In the embodiment, the moving coil 1200 and the second magnetic coil 1400 may have a magnetic force having the same polarity when receiving the pulse current. Thus, when a magnetic force is simultaneously formed in the moving coil 1200 and the second magnetic coil 1400, a repulsive force may be generated between the moving coil 1200 and the second magnetic coil 1400, and thus the moving coil 1200 and the plunger 1100 may be moved in the direction approaching the chemical solution chamber 100.

Although not illustrated in FIGS. 15 and 16, the drug injection device according to the ninth embodiment may include the discharge part 200, the chemical solution storage part 400, and the check valve 500 according to the sixth embodiment. Thus, when the moving coil 1200 and the plunger 1100 are moved in the direction approaching the chemical solution chamber 100, the chemical solution 10 stored in the chemical solution storage part 400 may be injected into the chemical solution chamber 100 through the check valve 500 by the negative pressure generated in the chemical solution chamber 100 after the chemical solution 10 is discharged from the chemical solution chamber 100 through the discharge part 200.

The moving coil current applying part 1500 may be electrically connected to the moving coil 1200 to apply the pulse current to the moving coil 1200.

The first magnetic coil current applying part 1600 may be electrically connected to the first magnetic coil 1300 to apply the pulse current to the first magnetic coil 1300.

The second magnetic coil current applying part 1700 may be electrically connected to the second magnetic coil 1400 to apply the pulse current to the second magnetic coil 1400.

The moving coil current applying part 1500, the first magnetic coil current applying part 1600, and the second magnetic coil current applying part 1700 may apply the pulse current under control of the controller 600.

Hereinafter, an operation of the drug injection device according to the ninth embodiment of the present disclosure will be described. The following process may be performed by the controller 600.

First, the moving coil current applying part 1500 and the first magnetic coil current applying part 1600 simultaneously apply the pulse currents to the moving coil 1200 and the first magnetic coil 1300.

As a result, as a repulsive force is generated between the moving coil 1200 and the first magnetic coil 1300, the moving coil 1200 and the plunger 1100 may be moved in a direction approaching the chemical solution chamber 100. In this case, as the plunger 1100 moves in the direction approaching the discharge part 200 and applies pressure for discharging the chemical solution 10 stored in the chemical solution chamber 100 to the discharge part 200, the chemical solution 10 stored in the chemical solution chamber 100 may be discharged to the discharge part 200.

Next, the moving coil current applying part 1500 and the second magnetic coil current applying part 1700 simultaneously apply the pulse currents to the moving coil 1200 and the second magnetic coil 1400.

As a result, as a repulsive force is generated between the moving coil 1200 and the second magnetic coil 1400, the moving coil 1200 and the plunger 1100 may be moved in a direction spaced apart from the chemical solution chamber 100.

According to the present disclosure, in the drug injection device according to the embodiment of the present disclosure, the pressure chamber and the elastic film are not required, and thus a structure thereof may be simplified.

Further, in the drug injection device according to the embodiment of the present disclosure, the temperature of the chemical solution stored in the chemical solution chamber may be adjusted so that the chemical solution injected from the chemical solution chamber into the deep skin may transfer heat and cold to the deep skin.

Hereinabove, the embodiments of the present disclosure have been described with reference to the accompanying drawings. However, those skilled in the art to which the present disclosure pertains may understand that the present disclosure may be implemented in other specific forms without changing the technical spirit or essential features thereof. Therefore, it should be understood that the embodiments described above are illustrative but not limiting in all aspects.

## Claims

1. A drug injection device comprising:
a chemical solution chamber in which a chemical solution is stored;
a discharge part which is provided in the chemical solution chamber and through which the chemical solution is discharged; and
a laser supply part configured to supply a pulsed laser to the chemical solution to generate bubbles,
wherein the bubbles induce volume expansion of the chemical solution and are dissipated to generate shock waves that push the chemical solution to the discharge part.

2. The drug injection device of claim 1, wherein the chemical solution is a liquid, a sol, or a liquid material in which the bubbles are generated by the pulsed laser.

3. The drug injection device of claim 1, further comprising:
a chemical solution storage part configured to store the chemical solution to be supplied to the chemical solution chamber; and
a check valve configured to allow the chemical solution stored in the chemical solution storage part to be transferred only to the chemical solution chamber,
wherein the chemical solution stored in the chemical solution storage part is injected into the chemical solution chamber through the check valve by a negative pressure generated in the chemical solution chamber after the chemical solution is discharged through the discharge part.

4. The drug injection device of claim 3, further comprising:
a temperature sensor configured to measure a temperature of the chemical solution stored in the chemical solution chamber;
a flow rate sensor configured to measure a flow rate of the chemical solution discharged through the discharge part;
a pressure sensor configured to measure a pressure of the chemical solution stored in the chemical solution chamber; and
a controller configured to forcibly open the check valve so that the chemical solution stored in the chemical solution storage part is supplied to the chemical solution chamber when at least one of the temperature value measured by the temperature sensor, the flow rate value measured by the flow rate sensor, and the pressure value measured by the pressure sensor exceeds a set reference value.

5. The drug injection device of claim 1, wherein the chemical solution includes a filler.

6. The drug injection device of claim 1, further comprising:
a chemical solution guide provided in the discharge part and configured to guide the chemical solution discharged through the discharge part to be sprayed in form of fine particles.

7. The drug injection device of claim 6, wherein the chemical solution guide includes:
a guide body provided in the discharge part and configured to reduce a cross-sectional area of the discharge part; and
a guide groove formed in a spiral shape on an outer circumferential surface of the guide body, and
wherein the chemical solution discharged through the discharge part flows along the guide groove to generate a vortex and is sprayed in the form of fine particles.

8. The drug injection device of claim 3, further comprising:
a temperature adjusting part configured to heat or cool the chemical solution stored in the chemical solution chamber so that a temperature of the chemical solution stored in the chemical solution chamber is maintained at a set temperature.

9. The drug injection device of claim 8, wherein the temperature adjusting part includes:
a Peltier element provided in the chemical solution chamber and configured to emit cold or heat; and
a current supply part configured to adjust a direction of a current applied to the Peltier element so that the Peltier element emits cold or heat according to the set temperature.

10. The drug injection device of claim 8, wherein the chemical solution chamber includes an insulating material.

11. The drug injection device of claim 8, further comprising:
an auxiliary temperature adjusting part provided in the chemical solution storage part and configured to heat or cool the chemical solution stored in the chemical solution storage part so that the chemical solution stored in the chemical solution storage part is maintained at the set temperature.

12. The drug injection device of claim 9, wherein the temperature adjusting part includes:
an auxiliary Peltier element provided in the chemical solution storage part and configured to emit cold or heat; and
an auxiliary current supply part configured to adjust a direction of a current applied to the auxiliary Peltier element so that the auxiliary Peltier element emits cold or heat according to the set temperature.

13. The drug injection device of claim 1, further comprising:
a plunger provided to reciprocate in the chemical solution chamber and approaching the discharge part to apply a pressure for discharging the chemical solution stored in the chemical solution chamber to the discharge part;
a moving coil provided in the plunger;
a first magnetic coil provided on one side of the chemical solution chamber and configured to apply a magnetic force for bringing the moving coil closer to the discharge part; and
a second magnetic coil provide on an opposite side of the chemical solution chamber and configured to apply a magnetic force for spacing the moving coil apart from the discharge part.

14. The drug injection device of claim 13, further comprising:
a moving coil current applying part configured to apply a pulse current to the moving coil;
a first magnetic coil current applying part configured to apply a pulse current to the first magnetic coil; and
a second magnetic coil current applying part configured to apply a pulse current to the second magnetic coil.
